# EUROPÄISCHE PATENTSCHRIFT

(11) **EP 1 309 272 B1**
(45) Veröffentlichungstag und Bekanntmachung des Hinweises auf die Patenterteilung: **16.12.2009**
(21) Anmeldenummer: 01967134.6
(22) Anmeldetag: 09.07.2001
(51) Int. Cl.: A61B 5/021

(54) **SYSTEMELEMENTE ZUR DRUCKMESSUNG IN EXTRAKORPORALEN KREISLÄUFEN**
SYSTEM ELEMENTS FOR MEASURING PRESSURE IN EXTRACORPOREAL CIRCUITS
ELEMENTS DE SYSTEME POUR MESURER LA PRESSION DANS DES CYCLES EXTRACORPORELS

(30) Priorität: 08.07.2000 DE 10032616
(43) Veröffentlichungstag der Anmeldung: 14.05.2003
(73) Patentinhaber: Überreiter, Andreas, 63743 Aschaffenburg (DE)
(72) Erfinder: ÜBERREITER, Andreas, 63743 Aschaffenburg (DE)
(86) Internationale Anmeldenummer: PCT/EP2001/007879
(87) Internationale Veröffentlichungsnummer: WO 2002/003854

(56) Entgegenhaltungen:
- EP-A- 0 878 628
- WO-A-97/39679
- DE-A- 4 419 593
- US-A- 5 000 049
- US-A- 5 722 399

## Beschreibung

Die Erfindung betrifft ein Systemelement zur lösbaren abgedichteten Verbindung eines Meßwertaufnehmers mit einem Fluidsystem, mit einer mit dem Fluidsystem durchströmbar verbindbaren Messkammer, wobei die Messkammer in einem Gehäuse gebildet ist und ein Teil der Wandung der Messkammer durch eine Membran gebildet ist, die wesentlich nachgiebiger ist, als der übrige Teil der Wandung der Messkammer.

Solche Verbindungselemente sind in der medizinischen Technik unter der umgangssprachlichen Bezeichnung "Dom" oder "Druckdom" bekannt, was von der kuppelförmigen Ausbildung der Messkammer herrührt, bzw. unter der Bezeichnung "Transducer", worunter ein Messwandler in einem geeigneten Gehäuse verstanden wird, der die üblicherweise über die Membran des Druckdomes übertragenen Drücke und Druckänderungen in ein elektrisches Signal umsetzt. Sie dienen dazu, die Messung von Drücken in Flüssigkeiten bei der Untersuchung und Behandlung von Menschen und Tieren vorzugsweise über elektronische Diagnose- und Überwachungsgeräte zu ermöglichen.

### Beschreibung des Standes der Technik

Für die Drucküberwachung beim Spülen von Körperhöhlen ist beispielsweise in DE 42 19 888 A1 ein Durchfluss-Druckwandler mit einem solchen Verbindungselement beschrieben, der dem vorgesehenen Einsatzgebiet entsprechend für einen großen Volumendurchsatz ausgebildet ist. Zugleich soll der Durchfluss-Druckwandler als Überdruckventil ausgebildet sein, durch das bei Überschreiten des durch die bauliche Auslegung des Durchfluss-Druckwandlers vorbestimmten Differenzdrucks auf der Druckseite einer Peristaltikpumpe zur Saugseite der Pumpe die Peristaltikpumpe kurzgeschlossen wird.

Zur Überwachung der hämodynamischen Parameter eines Patienten, insbesondere von Intensivpatienten, ist es heute üblich, neben der Aufzeichnung eines EKG's auch die invasiven Drücke in das Patienten-Monitoring, daß heißt, die Zustandsüberwachung der vitalen Körperfunktionen des Patienten, aufzunehmen. Es werden je nach Überwachungsgrad zwischen einem bis vier Drücken (arteriell, pulmonalarteriell, LAP und venös) gemessen.

Hierzu wird ein Katheter mit einem eingebundenem Monitoring-Set benutzt. Die Positionierung der Endöffnung des Katheters definiert den Messpunkt im Körper des Patienten. Als Monitoring-Set wird eine Zusammenstellung derjenigen meist aus hygienischen Gründen zur Einmalverwendung bestimmten Teile bezeichnet, die die Verbindungen zwischen dem Patienten und dem sogenannten Monitor herstellen. Als Monitor bezeichnet man das elektronische Überwachungs- und Aufzeichnungssystem, mit dem die entsprechenden Messdaten ausgewertet und angezeigt werden, und das im Bedarfsfalle entsprechende Alarmsignale ausgibt, wenn Messdaten vorgegebene Sollbereiche verlassen.

Am Beispiel einer Einschwemmkatheteruntersuchung findet sich eine allgemeine Beschreibung hierzu in Buchwalsky, Rainer: Einschwemmkatheter: Technik, Auswertung u. prakt. Konsequenzen (Beiträge zur Kardiologie, Bd. 29); Erlangen: perimed Fachbuch-Verlagsgesellschaft, 1985, Seiten 106-109.

Das am Katheter zu befestigende Monitoring-Set besteht dabei aus einem unbelüfteten Infusionsgerät zur Zuführung von Infusionslösungen zu dem Patienten, einem Spülsystem, welches eine kontinuierliche Spülrate von üblicherweise 3 ml/h an der Katheterspitze zu Vermeidung von Okklusion durch Thromben gewährleistet, gegebenenfalls mit Schnellspülfunktion für Sonderfälle und einem Druckdom. Der Druckdom überträgt über seine flexible Membran das Drucksignal auf einen wiederverwendbaren Transducer (Drucksensor). Ein solcher Druckdom wurde bisher mit einer Schraub- oder Bajonettverbindung auf einem solchen Transducer befestigt (siehe dazu DE 42 19 888 A1, Spalte 3, Zeilen 28 bis 30), oder mittels Schnapphaken (vgl. WO 99/37983).

Beispiele solcher Druckdome finden sich auf einem Informationsblatt "Disposable Transducer Dornes" der Fa. SMP Specialty Medical Products, Dallas, Texas, US, mit Bezug auf die Modelle 078 bis 082. Ein typischer Transducer (Drucksensor) ist beispielsweise in einem Prospektblatt der Fa. SensoNor a.s, Horten, N0, Edition 1/95, zu dem Produkt SensoNor 840 beschrieben.

Weitere Elemente eines Monitoring-Set's sind die Druckschläuche (farbig gekennzeichnet) und eventuell ein Dreiwegehahn, um Medikamente zuführen zu können, oder ein Blutentnahmesystem zur Entnahme von Blut für weitere Untersuchungen.

Ein spezielles Problem besteht dabei darin, die mit dem Blutsystem in Verbindung stehenden Teile des Monitoring-Sets zu entlüften. Die Problematik des Entlüftens solcher Systeme ist den Anwendern allgemein bekannt. Besonders leicht fangen sich Luftblasen beim Befüllen des Systems (meist mit physiologischer Kochsalzlösung) im Dom, d.h. in seiner kuppelartigen Messkammer oberhalb der Membrane. Die dort gefangenen Luftblasen sind wegen der den Gasen eigenen großen Elastizität im Gegensatz zu den praktisch inkompressiblen Flüssigkeiten eine Sperre bei der Übertragung von Druckfrequenzen von mehr als einigen Hertz. Dadurch wird die Übertragung der Druckänderung auf die Membran und damit den darunterliegenden Druckmeßaufnehmer und somit die Darstellung der Druckkurven auf dem Monitor deutlich verfälscht.

Durch offenkundige Vorbenutzung der SMP Specialty Medical Products, Dallas, Texas, US, ist unter der Typenbezeichnung 081 ein Verbindungselement der eingangs erwähnten Art bekannt. Dieser "Dom" soll zu dem Transducer Hewlett Packard 1290 Quartz passen und über eine Bajonettverbindung an diesem befestigbar sein. Zur Verbindung von Ein- und Austrittskanal mit den Schläuchen eines Momtonng-Sets dienen verrriegelbare Luer-Lock-Verbindungen mit losem Gewindeteil bzw. mit Außen-Vollgewinde, wie sie beispielsweise in DIN 13 090 Teil 2 angegeben sind.

Das bekannte Verbindungselement ist aus einem glasklaren Kunststoff hergestellt. Die Messkammer dieses Verbindungselementes ist sehr groß und hat insbesondere einen großen Durchmesser von ca. 23 mm. Dabei ist die Decke der Messkammer zugleich die Oberseite des Gehäuses. Diese Decke und Oberseite des Gehäuses ist als Vergrößerungslinse plankonvex ausgebildet Dadurch soll erreicht werden, daß auch kleine Luftblasen in der Messkammer möglichst von dem medizinischen Pflegepersonal sicher erkannt werden.

Einmaltransducer, die den Druckmeßsensor in einem Durchflussgehäuse enthalten, sind daher in der Durchflusskammer einfach röhrenförmig gestaltet, um eben dieses Fangen von Luftblasen zu vermeiden. Sie haben aber den Nachteil, daß die wertvolle Elektronik im Einmalartikel integriert ist und daher bei jedem Wechseln des Monitoring-Set's weggeworfen wird und mit zu entsorgen ist. Unter Beachtung der Hygieneanforderungen muß ein solcher Austausch spätestens jeden zweiten Tag erfolgen. Dies bringt nicht nur den Nachteil mit sich, daß die noch funktionsfähige Elektronik mit jedem Wechsel ersetzt wird und entsprechende Kosten mit sich bringt, sondern auch das Vorhandensein von elektronischen Bauelementen eine zusätzliche besondere und damit kostenintensive Behandlung als Elektronikschrott bei der Entsorgung erfordert.

Aus diesem Grund sind zumindest in Europa wieder mehrfach verwendbare Dom-Systeme auf dem Vormarsch. Die wertvolle Elektronik, insbesondere der Drucksensor, befindet sich in einem speziellen Gehäuse. Ein solches Teil wird üblicherweise als Transducer bezeichnet. Ein oder mehrere Transducer werden in einer speziellen Halteplatte integriert. Die Halteplatte wird mittels einer Klemm- oder Schraubvorrichtung z.B. an einem Infusionsständer befestigt. Von den Transducern in der Halteplatte werden die Druckmessdaten über ein oder mehrere Kabel zum Monitor übertragen.

Dieses Problem wird auch nicht gelöst durch ein Verbindungselement der eingangs erwähnten Art, wie es in DE 35 25 536 A1 beschrieben ist. Dort wird zur Vermeidung von Beschädigungen insbesondere der Membran des Verbindungselementes eine Befestigung vorgeschlagen, bei der eine gegenseitige Verdrehung von Verbindungselement und Transducer nicht erforderlich ist. Dazu sollen an zwei Stellen des Umfangs des Gehäuses des Verbindungselementes zusätzliche Befestigungselemente vorgesehen werden, wie schwenkbar montierte Klammern oder nach Art von Schließhaken.

Dort wird vorgeschlagen, diese Befestigungselemente oder Fortsätze zur Handhabung der Befestigungselemente nach unten über die Membran hinausragen zu lassen. Dadurch soll eine visuelle Kontrolle möglich sein, daß eine Verriegelung der vorgesehenen Befestigungselement auch durchgeführt wurde. Ferner soll dies die Handhabung beim Entfernen des Verbindungselementes von dem Transducer vereinfachen. Eine Kombination mit in Halteplatten befestigten Transducern ist dadurch jedoch praktisch ausgeschlossen.

Weiterhin sollen gemäß der Lehre dieser Druckschrift die Gehäuse von Druckdom und Transducer sich direkt berühren (siehe dort, Spalte 9/10), wodurch zwar die Membranen von Druckdom und Transducer "satt aufeinander" liegen sollen, eine Vorspannung der Membranen soll jedoch nachteilig sein und vermieden werden (a.a.0., Spalte 4, Zeilen 21 ff.).

Die EP 0 701 830 A1 beschreibt eine Vorrichtung und ein Verfahren vorzugsweise zur Anwendung im Bereich der Druckmessung in blutführenden Leitungen bei Dialyseeinrichtungen, bei der Hämofiltration und der Hämodiafiltration. Dazu soll der Druck indirekt über eine Gassäule (Luft) gemessen werden, die über eine in einem Gehäuse befindliche elastische Membran mit der Flüssigkeitssäule in einer Leitung in Verbindung steht. Der Druck der Gassäule wird über geeignete und übliche Sensoren (Druckmeßdosen) erfaßt. Um den aufgrund der mechanisch begrenzten Auslenkbarkeit der Membran und die Kompressibilität der Gassäule bedingte Beschränkung des Meßbereiches zu erweitern, wird eine Vorrichtung und ein Verfahren vorgeschlagen, mit dem die Gasmenge in dem gasführenden Teil der Messeinrichtung in Abhängigkeit von dem zu messenden Gasdruck erhöht oder erniedrigt wird und dadurch eine Beweglichkeit der Membran und damit die Weitergabe von Druckänderungen erhalten bleibt.

Die Veränderung der Gasmenge erfolgt über Pumpeinrichtungen in Form einer über eine Steuerung betätigten peristaltischen Pumpe. Durch die Kompressibilität des gemäß der EP-Anmeldung zwischenzuschaltenden Gasvolumens wird eine Art akustischer Tiefpass gebildet, der schnelle Druckänderungen oder höherfrequente Druckschwingungen dämpft bzw. unterdrückt. Das System nach der Druckschrift ist praktisch nur für Messung statischer Drücke geeignet oder zur Überwachung von Mittelwerten, wobei die Bildung des Druckmittelwertes durch die Dämpfungscharakteristik des Systems einstellbar ist, z.B. über das zwischen Membran und Druckaufnehmer geschaltete Gasvolumen. Für Anordnungen solcher Art ist durch Vorbenutzung eine obere Grenzfrequenz von etwa 0,1 Hz für die Erfassung von Druckänderungen bekannt.

Die Schrift beschreibt ferner im Detail ein Verfahren mit Abwandlungen, wie unter Verwendung einer elektronischen Steuerung durch sukzessive Erhöhung und Erniedrigung der Gasmenge in dem gasgefüllten Teil des Systems die gewünschte Einstellung der Lage der Membran und damit des Messbereiches erfolgen kann, ohne die Lage der Membran mechanisch zu erfassen oder beobachten zu müssen.

In der Schrift ist offenbart eine Druckmesseinrichtung mit einem für Veränderungen eines Gasdruckes empfindlichen Druckaufnehmer (38), der über eine luftgefüllte Leitung (37) mit einem Gehäuse (30) verbunden ist. Letzteres ist durch eine biegsame Membran (33) unterteilt in zwei Kammern (31, 32), wobei die erste Kammer (31) zumindest eine Öffnung zum Zuführen von Flüssigkeiten aufweisen soll.

Die zweite Kammer (32) soll über einen Rohranschluss gasdicht mit der Leitung (37) verbunden und damit an den Druckaufnehmer (38) angeschlossen werden können.

In DE 29 30 869 C2 wird beschrieben eine Druckmesskapsel für die Befestigung an einem Messwandler, die ein Gehäuse aufweist, in dem innerhalb eines ringförmigen Wulstes ein Hohlraum gebildet ist, der durch eine auf den ringförmigen Wulst geklebte Membran geschlossen ist. Über zwei Anschlußröhren soll der Hohlraum mit einer Flüssigkeit oder einem Gas gefüllt werden können.

Wesentlicher Gegenstand der Beschreibung ist die Ausbildung von Fortsätzen eines zylindrischen Gehäusekörpers der Druckmesskapsel zur Bildung einer Renkverbindung mit entsprechend ausgebildeten Gegenstücken an einem Messwandler. Insbesondere wird hervorgehoben eine elastische Ausbildung der Fortsätze zur Bildung definierter rastbarer Endstellungen der Partner der Renkverbindung, wobei Druckaufnehmer und Druckmesskapsel zur Herstellung der mechanischen Verbindung gegeneinander verdreht werden müssen. Durch die federelastische Ausbildung der druckmesskapselseitigen Teile der Renkverbindung sollen Fertigungstoleranzen ausgleichbar und eine rastbare Endstellung von Druckmesskapsel zu Druckaufnehmer erreichbar sein.

Durch die Raststellung soll zugleich eine definierte Vorspannkraft auf die Druckaufnehmer ausgeübt werden, wobei sich die Erfinder vorstellen, daß diese so gleichbleibend reproduzierbar mit verschiedenen Druckaufnehmern ausfallen soll, daß ein Nullpunktabgleich der verwendeten Auswerteelektronik nicht mehr erforderlich sei.

Die zitierte Druckschrift beschreibt eine Renkverbindung als besonders vorteilhaft, deren bekannte Nachteile insbesondere eine reibende Relativbewegung zwischen den zu verbindenden Partnern der Renkverbindung ist.

Das Europäische Patent EP 0 330 891 B1 beschreibt eine Anordnung zum Übertragen des Druckes eines Fluides auf ein anderes Fluid. Dazu wird ein längliches Gehäuse vorgeschlagen, dessen Innenraum in Form eines Rotationsellipsoids durch eine flexible Membran in zwei Räume aufgeteilt wird, wobei diese beiden Räume derart angeordnet sein können, daß sie jeweils benachbarte Bereiche des Innenraums bilden, oder auch konzentrisch. Einer der Räume soll mit jeweils einer Einlaß- und einer Auslassöffnung versehen sein, um mit einem ersten Fluid durchströmt zu werden, wie beispielsweise Blut.

Der zweite Raum ist mit einer einzigen Öffnung versehen, über die ein in dem zweiten Raum einbringbares Fluid mit z.B. einer externen Druckmesseinrichtung verbunden sein soll, um etwa den Druck des Blutes, der durch den ersten Raum strömt, zu messen.

Als erfindungswesentlich wird beschrieben und beansprucht, die Membran in einem ungespannten oder sogar gefalteten Zustand in das Gehäuse einzubringen, wodurch sich die dortigen Erfinder eine Verbesserung der Messmöglichkeiten des Druckes und insbesondere der Messung auch negativer Drücke erhoffen, ohne dass hierzu nähere Angaben gemacht werden.

In WO 97/39679 ist eine Kopplung einer Art Druckdom mit einem Transducer beschrieben, wobei die Messkammer des "Druckdomes" jedoch nicht durch eine Membran gegenüber der Umgebung abgeschlossen ist, sondern durch ein isolierendes Gel. Bei der Montage von Druckdom und Transducer soll die Fließfähigkeit des Gels das Herausdrücken von Luft zwischen Druckdom und Transducer durch Entlüftungskanäle ermöglichen.

In US 4,562,845 ist eine Verschraubung beschrieben, die zu einer dichten Koppelung eines Druckdomes mit einem Transducer führt. Da die dort beschriebene Vorrichtung Teil des ebenfalls dort beschriebenen Systems zur Überwachung von anderen Transducern auf Fehlfunktion sowie zur Erfassung von Luftblasen in Blutdrucküberwachungssystemen sein soll, weist der dort beschriebene Druckdom keine Membran zur sterilen Abdichtung des Fluidsystems gegenüber der Umgebung und dem Transducer auf.

Aus US 4,462,409 ist ein Druckdom bekannt, der allerdings nicht zum Einschleifen in einen extrakorporalen Kreislauf oder zur Durchströmung mit einer Infusionslösung vorgesehen ist, sondern als Abschluss einer Stichleitung, die hydraulisch über ein Infusionssystem mit dem Kreislauf eines Patienten zur Druckübertragung gekoppelt werden kann. Die Messkammer dieses Druckdoms ist über eine Membran von einem Transducer getrennt. Der Transducer umfasst ein zweiteiliges Gehäuse, wobei ein erstes Gehäuseteil (dort 53) eine Verbindungsfläche zur Anlage der Membran des Druckdomes aufweist. Auf dieser Verbindungsfläche des Gehäuseteils ist eine Rippe (63a) vorgesehen, die einen festen Sitz einer Wulst der Membran in einer Nut in dem Gehäuse und damit eine sichere Abdichtung der Messkammer sicherstellen soll.

Allerdings ist der Druckdom mit dem Gehäuseteil des Transducers durch Schweißen fest verbunden (a.a.0., Spalte 4, Zeilen 60-67), so dass eine Anordnung mit einem Einwegdom und einem wiederverwendbaren Transducer nicht möglich ist.

Die US 4,9200,972 beschreibt ein System aus einem Einmaldom und einem wiederverwendbaren Transducer, bei der die Messkammer im Dom sowie der Transducer jeweils über eine Membran abgeschlossen sind. Im übrigen beschäftigt sich die Lehre dieser Druckschrift mit dem Ersatz von Öl zur Druckübertragung innerhalb des Transducers durch ein Gel, dass erst nach Einbringen in flüssiger Form in das Transducergehäuse durch Aufheizen des Transducers auf 65° C für vier Stunden zu der gewünschten Gelform aushärtet. Durch diese Anordnung soll die oberer Grenzfrequenz eines Transducers vergrößert werden.

Aus US 5,551,300 ist ein Set aus einem Einweg-Druckdom und einem wiederverwendbaren Transducer bekannt, bei dem sowohl die Messkammer des Druckdoms, als auch ein flüssigkeitsgefüllter Messraum des Transducers durch eine flache, auf die jeweiligen Gehäuse aufgeklebte Membran abgeschlossen sind. Dabei ist bei dem Transducer ein Druckausgleich des flüssigkeitsgefüllten Messraumes des Transducers vorgesehen derart, dass der Messraum in Strömungsverbindung mit einem Ausgleichsgefäß steht, das über eine elastische Membran gegenüber der Umgebung abgeschlossen ist.

Das Flüssigkeitssystem soll dabei mit einem leichten Überdruck befüllt sein, um einen Kontakt der beiden druckübertragenden Membranen und damit die Einsatzfähigkeit des Systems sicherzustellen. Um während der Messung einen Druckausgleich im Flüssigkeitssystem des Transducers zu verhindern, was Voraussetzung für eine Druckmessung ist, wird vorgeschlagen, die Transducermembran etwas abstehen zu lassen und eine Verbindung der flüssigkeitsgefüllten Messkammer des Transducers mit dem Druckausgleichsbehälter über ein Loch auf der Stirnseite des Transducers vorzusehen, das ebenfalls von der Transducermembran überdeckt wird. Durch Verbindung mit dem Druckdom wird die Membran an das Gehäuse des Transducers mechanisch angelegt und dadurch die Ausgleichsöffnung verschlossen, so dass lediglich die in der Messkammer des Transducers verbleibende Flüssigkeit eine Signalübertragung auf einen Piezosensor dämpfen kann.

Aus DE 44 19 593 A1 ist eine Vorrichtung zum Messen des Drucks eines Mediums bekannt, insbesondere zur Druckmessung in extrakorporalen Blutkreisläufen, z. B. einem Dialysesystem, bei dem ein Wegwerfelement vorgesehen ist, das eine Messkammer enthält und über zwei Schlauchanschlüsse verfügt, wobei die Messkammer durch eine Membran geschlossen sein soll, die in eine umlaufende Nut eingelegt und mittels eines metallischen Spannringes oder durch Verklebung dort befestigt sein soll. Als besonders zweckmäßig wird dort hervorgehoben, dass um den Teil der Messkammer herum, der mit der Membran in Verbindung steht, ein umlaufender Wulst insbesondere durch einen 0-Ring gebildet wird, der die Membran im Bereich der Verbindung zur Messkammer über die Oberfläche des Elementes hinaus anhebt. Dadurch soll es möglich sein, eine gute Ankoppelung der Membran an einen Druckmessaufnehmer zu erhalten, wenn das Element in eine Schublade des Messsystems eingelegt und der Druckaufnehmer vorzugsweise pneumatisch oder über eine Spindel zu dem Element hin verfahren wird.

Gegenüber den weiter vorne beschriebenen Drucksystemen aus dem Stand der Technik ist allerdings hervorzuheben, dass sich das Element nicht selbst entlüftet und insbesondere im Bereich zwischen der Verbindungsöffnung zur Messkammer und dem umgebenden Wulst unter der Membran zahlreiche Spalte und Rücksprünge verbleiben, in denen sich bei der Befüllung des Systems leicht Luftblasen sammeln können. Wie bereits weiter oben ausgeführt, ist dies nicht nur hinsichtlich der Qualität der Druckübertragung bzw. der Blutdruckmessung nachteilig, sondern es besteht auch das erhebliche Risiko einer Verklottung des Blutes bzw. der Bildung von Blutgerinnseln, was insbesondere bei extrakorporalen Blutkreisläufen lebensgefährlich für den Patienten werden kann, wenn solche Blutgerinnsel nicht vor der Einspeisung des Blutes in den Körper abgefangen werden.

Entsprechend enthält diese Veröffentlichung keine näheren Angaben zum Messbereich des Systems oder der Qualität der Messungen, insbesondere zum Grenzfrequenzbereich, der Rückschlüsse auf Dämpfung des Systems durch in der Messkammer verbliebene Luftblasen zuließe.

Weiterhin erfordert die Vorrichtung gemäß der DE 44 19 593 A1 einen enorm hohen Montageaufwand für das Messelement, insbesondere im Hinblick auf die Montage der Membran, was zusätzlich zu einem großen Aufwand hinsichtlich der Überprüfung der Qualität der Montage aus Gründen der Produkthaftung führt. Weiterhin kann die Vielzahl der Teile und insbesondere die zwischen Membran und 0-Ring gebildeten Hohlräume zudem zu Problemen bei der Sterilisation führen. Im Hinblick auf den Montage- und Qualitätssicherungsaufwand führt dies zu derart hohen Kosten, dass ein solches Messsystem für den Einweggebrauch nicht akzeptabel ist.

Weiterhin erfordert die entsprechende Anordnung des Druckmesssensors einen enorm hohen apparativen Aufwand, insbesondere im Hinblick auf die Verfahreinrichtung für den Sensor und die von der Verfahrstrecke abhängige Kalibrierung des Sensors, so dass ein solches System aufgrund des Handhabungsaufwandes und der enormen Kosten für den klinischen Alltag ungeeignet ist.

Aus DT 21 29 670 A ist eine sogenannte Unterdruckdose bekannt, bei der in einer festen Metalldose eine elastische Membran angeordnet ist, wobei alls erfindungswesentlich die Ausbildung der Membran und eine Befestigung an einem Stößel beschrieben ist, so dass die Membran bei der Herstellung auf einer Seite der Dose anliegt und nach Vorspannung durch eine Feder in Abhängigkeit von dem absoluten Luftdruck, mit der die Membran auf einer Seite beaufschlagt wird, eine Stellarbeit über den Stößel verrichten und insbesondere in Abhängigkeit von der Vorspannung über die Feder dabei einen beträchtlichen Hub ausführen kann. Diese Einrichtung ist jedoch nicht dazu vorgesehen, durchströmbar zu sein und aufgrund der Ausbildung und Zielsetzung gänzlich ungeeignet zur Messung von Drücken, insbesondere in extrakorporalen Kreisläufen.

Aus DE 93 17 751 U1 ist ein Druckanzeigegerät bekannt, dass bei Erreichen eines bestimmten vorgegebenen Druckwertes durch einen unter einer durchsichtigen Scheibe sichtbaren Farbumschlag von grün auf rot oder umgekehrt anzeigt und/oder über einen Stößel einen elektrischen Schalter oder Taster betätigen kann. Hierzu wird im Zwischenraum zwischen einer Membran und einem Messgehäuse eine Flüssigkeit eingeschlossen. Wird ein Grenzdruck auf der anderen Seite der Membran, die dem Verdampfungspunkt der Flüssigkeit entspricht, unterschritten, verdampft die Flüssigkeit und die Membran führt zusammen mit einem daran befestigten Stößel einen schlagartigen Hub aus, so dass mit dem Stößel ein Warntaster oder dergleichen betätigt werden kann. Durch eine entsprechende Einfärbung der Flüssigkeit wird die Durchsicht auf eine signalfarbene Scheibe auf der Membran behindert, die bei Verdampfen der Flüssigkeit schlagartig sichtbar wird und damit eine Signalwirkung entfalten soll.

Es ist ohne weiteres ersichtlich, dass diese Vorrichtung zur kontinuierlichen Messung von Drücken nicht geeignet ist.

Aus WO 99/37983 ist schließlich ein Druckdom bekannt, der besonders ausgebildet und geeignet ist zum Einsatz in extrakorporalen Blutkreisläufen, z.B. bei der Blutwäsche, der ferner besonders gut handhabbar ist durch eine lösbare Schnappverbindung zur Befestigung an einem Transducer.

Ein Verbindungselement gemäß der Präambel des Anspruchs 1 ist aus dem Dokument US-A-5 722 399 bekannt.

### Zusammenfassung der Erfindung

Der Erfindung liegt daher die Aufgabe zugrunde, ein Verbindungselement der eingangs erwähnten Art bereitzustellen, das hinsichtlich seiner auf die Anwendung bezogenen Zuverlässigkeit einerseits und seiner Handhabung andererseits verbesserte Eigenschaften aufweist, sowie eine sicherere und kostengünstigere Behandlung von Patienten ermöglicht.

Diese Aufgabe wird erfindungsgemäß gelöst durch ein Systemelement gemäß Anspruch 1.

Durch die erfindungsgemäße Ausbildung wird es erstmals möglich, Unterdrücke in Blutkreisläufen in größerem Umfang zu messen, als diese allein durch die Pumpleistung des Herzens entstehen können. Weiterhin ist es erstmals möglich, Unterdrücke direkt in Blutkreisläufen zu messen, die größer sind, als derjenige, bei dem rote Blutkörperchen in menschlichem Blut bei längerer Einwirkung platzen (Hämolyse). Weiterhin ist es erstmals möglich, so hohe Druckanstiegsgeschwindigkeiten zu erfassen, daß eine hinreichend schnelle Regelung von in Reihe geschalteten Peristaltikpumpen möglich ist, so dass die Dauer einer Unterdruckwirkung auf rote Blutkörperchen unter einer kritischen Grenze gehalten werden kann.

In der Medizintechnik werden in stark wachsendem Umfang zur lebenserhaltenden Funktion in kritischen Bereichen, wie Operationen am offenen Herzen, Multiorganversagen bei Infektionen oder Unfällen, Entgiftung durch Hämofiltration, zur Behandlung bei chronischer Niereninsuffizienz (Hämodialyse) sowie zur Gewinnung von Blutpräparaten durch Zellseparation extrakorporale Kreisläufe eingesetzt. Diese extrakoproralen Kreisläufe werden von üblicherweise bis zu 4 Pumpen, meist Peristaltikpumpen betrieben, wobei die Pumpen oft paarweise in Reihe geschaltet eingesetzt werden.

Die Pumpenköpfe von Maschinen von extrakorporalen Kreisläufen können die Pumpenschlauchsegmente nicht gleichmäßig genug bedienen, so dass es zu Über- und Unterdrücken im Schlauchsystem kommt, insbesondere wenn die stromabwärts angeordnete Pumpe schneller anläuft als die vorgeschaltete Pumpe. Dadurch entsteht unter Umständen nur für Sekundenbruchteile ein Unterdruck im Leitungsbereich zwischen den Pumpen. Gegen Unterdrücke sind die Blutbestandteile sehr empfindlich, die roten Blutkörperchen können platzen, es kommt zur Hämolyse. Um kritische Druckbereiche zu vermeiden, werden heute T-Stücke eingeschleift und über Stichleitungen und hydrophobierte Filter mit angeschlossenen Luftleitungen Drucktransducer auf PC-Boards eingesetzt. Diese Lösung ist aufgrund der elastischen Puffervolumina so langsam, dass die kritischen Druckbereiche zumindest kurzfristig nicht vermieden werden und so Unterdrücke bis - 300 mm Hg doch als Druckspitzen im Bereich von wenigen Sekunden auftreten.

Mit der erfindungsgemäßen Anordnung lassen sich Druckänderungen im Unterdruckbereich bis - 400 mm Hg bis zur Grenzfrequenz f_{G} von ca. 60 Hz erfassen. Dies wird unter anderem dadurch erreicht, dass die Membran des Druckdomes hinreichend luftfrei an die Transducermembran ankoppelbar ist und durch die erfindungsgemäße Ausbildung ein Eindringen von Umgebungsluft zwischen die gekoppelten Membranen hinreichend verhindert wird, so dass die Druckübertragung auf den Transducer auch im Unterdruckbereich hinreichend genau erfolgt.

In einer besonders bevorzugten Ausführungsform ist das erfindungsgemäße Systemelement dadurch gekennzeichnet, dass der Höhenunterschied der Außenwandung gegenüber der Innenwandung der Nut geringer ist als die durchschnittliche Dicke der Membran im Bereich außerhalb des umlaufenden Wulstes. Dadurch wird eine besonders gute Dichtwirkung mit dem Transducergehäuse erreicht, da erfindungsgemäß die Membran im Bereich des Wulstes nach Einsetzen des Wulstes in die Nut wenigstens etwa 0,1 mm, vorzugsweise zumindest etwa 0,3 mm über die Außenwandung der Nut herausragt.

Durch das Anpressen der Membran gegen die innere Wandung des Doms können sich eventuell im Bereich der Nut befindliche Luftbläschen nicht mehr negativ auswirken durch Dämpfung des Signals oder des Unterdruckes, auch können diese keine Verklottung des Blutes verursachen.

Zweckmäßigerweise ist die Membran aus einem EPDM hergestellt. Es kann aber auch besonders im Hinblick auf die Dichteigenschaften vorteilhaft sein, wenn die Membran aus einem TPE hergestellt ist, insbesondere, wenn die Membran aus einem TPE der Klasse SEBS hergestellt ist.

Für den erfindungsgemäßen Einsatz ist es besonders zweckmäßig, wenn die Membran bei einem Anpressdruck von 60 N gegen eine feingedrehte stählerne Oberfläche einen Unterdruck von 530 hPa gegen Luft dichtet.

Verfälschungen der Messung durch die Membransteifigkeit können weitgehend vernachlässigt werden, wenn die Membran innerhalb des Wulstes einen Durchmesser von wenigstens 12 mm aufweist.

Eine besonders gute Befestigung der Membran läßt sich erhalten, wenn der Wulst eine Breite von ca. 2 mm in Längserstreckung der Membran aufweist.

Einen guten Kompromiss zwischen Übertragungseigenschaften und Herstellungsaufwand sowie gute mechanische Haltbarkeit der Membran erhält man, wenn die Membran innerhalb des Wulstes eine Dicke von etwa 0,4 mm bis etwa 0,5 mm aufweist.

In einer zweckmäßigen Ausführungsform der Erfindung reicht der Meßbereich des Sensors wenigstens bis zu Differenzdrücken gegenüber der Umgebung von -350 mm Hg, bevorzugt umfaßt der Messbereich des Sensors wenigstens Differenzdrücke gegenüber der Umgebung von -400 mm Hg bis +3000 mm Hg.

Die für die Messung negativer Drücke erforderliche Dichtheit läßt sich erhalten, wenn der umgebende Bereich annähernd technisch eben und die Oberfläche des umgebenden Bereiches im wesentlichen kratzerfrei geschlichtet, vorzugsweise feingedreht, geschliffen oder poliert ist, insbesondere, wenn die Oberfläche des umgebenden Bereiches eine gemittelte Rauhtiefe R_{z} von nicht mehr als 20 µm (z.B. nach DIN 4768 T 1) und/oder die Oberfläche des umgebenden Bereiches eine maximale Rauhtiefe Rₘₐₓ von nicht mehr als 30 µm, vorzugsweise nicht mehr als etwa 20 µm aufweist.

Beim Zusammensetzen von Druckdom und Transducer läßt sich der Einschluss auch kleinster Luftpolster weitgehend vermeiden, wenn die Transducermembran gegenüber dem sie umgebenden Bereich geringfügig vorgewölbt ist, insbesondere, wenn die Transducermembran gegenüber dem sie umgebenden Bereich nicht mehr als 1 mm, vorzugsweise nicht mehr als 0,6 mm vorgewölbt ist.

Besonders gute Messeigenschaften lassen sich erhalten, wenn die Transducermembran aus einem RTV-Silicon gebildet ist. Es kann zur Verbesserung der Langzeitstabilität aber auch vorteilhaft sein, wenn die Transducermembran aus einem Polyurethan, vorzugsweise einem TPE-Polyurethan gebildet ist.

Die für die Messung negativer Drücke erforderliche Dichtheit lässt sich besonders sicher erhalten, wenn der umgebende Bereich eine Breite von wenigstens 2 mm aufweist.

### Kurzbeschreibung der Zeichnungen

Die Erfindung soll im folgenden anhand von in den Zeichnungen dargestellten Ausführungsbeispielen näher erläutert werden. Es zeigen:
- Fig. 1a-1e: einen Druckdom in verschiedenen Ansichten;
- Fig. 2: einen Transducer in Seitenansicht;
- Fig. 3: ein Set aus einem Druckdom und einem Transducer in teilweiser Schnittansicht; und
- Fig. 4: einen vergrößerten Ausschnitt der Darstellung gemäß Fig. 3 als Schnittansicht.

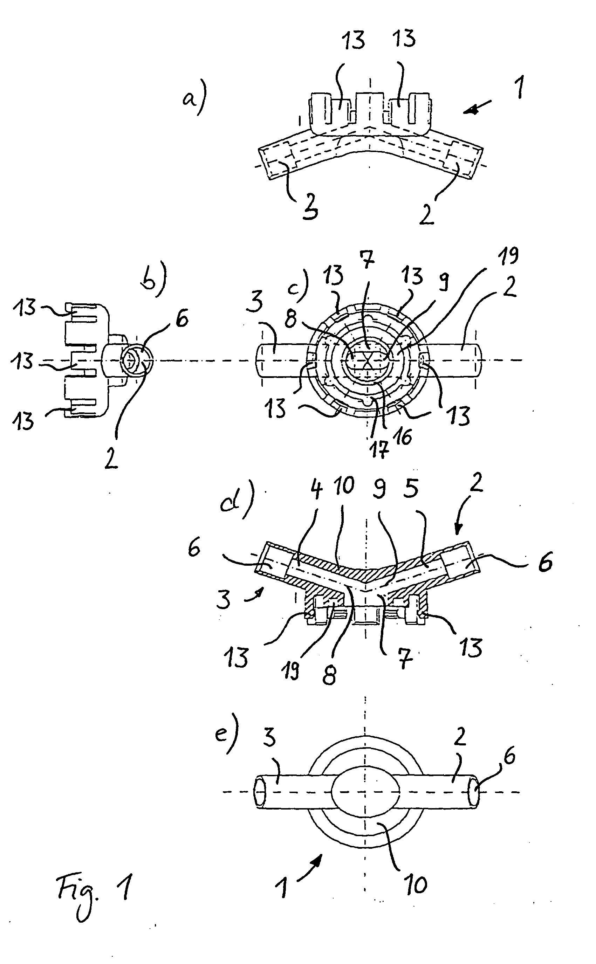

Das in Figur 1a bis 1e dargestellte Systemelement zur lösbaren abgedichteten Verbindung eines Messwertaufnehmers mit einem Fluidsystem in Form eines Druckdomes 1 weist zwei Anschlüsse 2, 3 zur Verbindung mit einem Infusionsgerät und mit einem Patienten, z. B. über einen eingeklebten Dreiwegehahn auf, oder zum Einschleifen in einen extrakorporalen Blutkreislauf, z.B. einem Dialyseapparat, einer Herz-Lungenmaschine oder einem Zellseparator. Die Anschlüsse 2, 3 weisen jeweils einen Kanal 4, 5 vorzugsweise mit einem Kegeldichtsitz, einer Einklebnut oder einem anderen in der Medizintechnik üblichen dichten Anschlusssystem 6 auf. Die Dimensionierung der Anschlüsse 2, 3 kann z.B. DIN 13090 entsprechen.

Mit dem Eintrittskanal 4 und dem Austrittskanal 5 ist eine Messkammer 7 über eine Eintrittsöffnung 8 und eine Austrittsöffnung 9 verbunden, so dass sich ein Strömungsweg von dem Eintrittskanal 4 durch die Messkammer 7 in den Austrittskanal 5 ergibt.

Die Messkammer 7 ist in einem Gehäuse 10 gebildet, das als einstückiges Spritzgussteil vorzugsweise aus einem transparenten Kunststoff, z. B. einem Polycarbonat, hergestellt ist. Ein Teil der Wandung der Messkammer 7 ist durch eine elastische Membran 11 (in Fig. 1 weggelassen) gebildet, z. B. aus einem EPDM, einem TPE, vorzugsweise aus einem TPE der Klasse SEBS oder sonst einem anderen geeigneten, gegen Blut und/oder Infusionslösung beständigen und physiologisch unbedenklichen Material. Somit besteht das Verbindungsteil vorzugsweise lediglich aus der Membran 11 und einem einstückigen Kunststoffspritzgussteil.

Der Werkstoff der Membran sowie dessen Verarbeitung, z.B. Spritzgießen, bei der Herstellung sollte eine Membran ergeben, die bei einem Anpressdruck von 60 N gegen eine feingedrehte stählerne Oberfläche einen Unterdruck von 530 hPa gegen Luft dichtet.

In Höhe der Membran 11 ist ferner eine Einrichtung zur mechanischen Kopplung des Verbindungselementes mit dem Messwertaufnehmer (Transducer 12) vorgesehen, die Teil einer lösbaren Einspreizverbindung ist, die gebildet wird durch krallenförmige Halteelemente in Form von Haken 13 zum Eingriff in eine entsprechende Nut oder Hinterschneidung 14 des Messwertaufnehmers 12 bzw. einer zugehörigen Befestigungseinrichtung.

Die Haken 12 sind durch federelastische Fortsätze des Gehäuses 10 gebildet und vorzugsweise sechs Stück gleichmäßig über den Umfang des Druckdomes 1 verteilt. Dabei ist die federelastische Verbindung von Haken 12 mit dem Gehäuse 10 so ausgebildet, dass eine Vorspannkraft von wenigstens etwa 60 N gehalten werden kann.

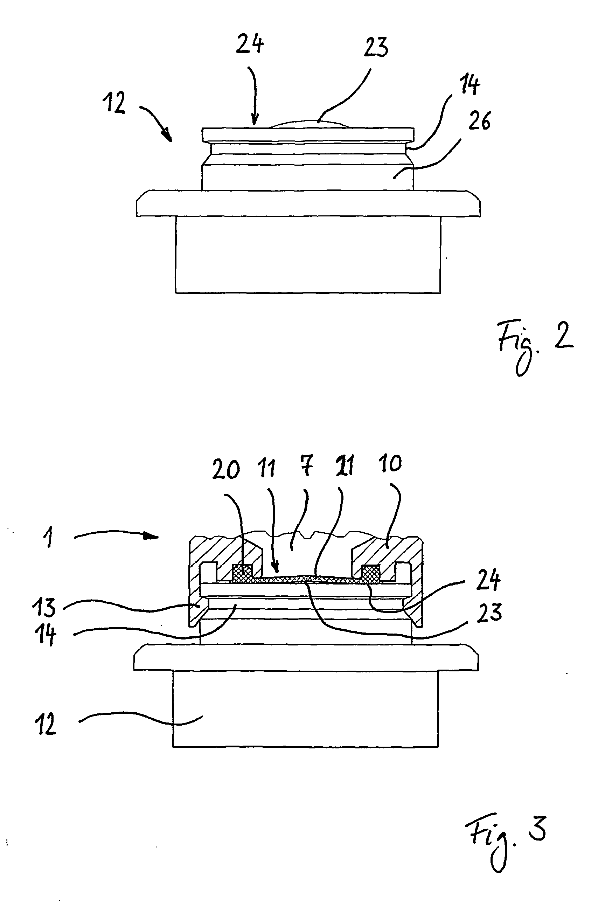

Wie in Fig. 3 gut zu erkennen ist, kann der Druckdom 1 durch einfaches Aufdrücken auf den Transducer 12 in einer Richtung annähernd senkrecht zu einer Ebene, die durch die Membran 11 aufgespannt wird, auf den Messwertaufnehmer 12 aufgesetzt werden.

Die Messkammer 7 ist radial außen durch einen Rand 15 bildenden Teil ihrer Wandung begrenzt, wobei Eintrittsöffnung 8 und Austrittsöffnung 9 an diesen Rand 15 anstoßen. Dazwischen und der Membran 11 gegenüberliegend ist eine Decke (nicht dargestellt) der Messkammer 7 durch einen Teil der Wandung gebildet.

Der Rand 15 wird nahe der Membran 11 durch eine Innenwand 16 gebildet, die zusammen mit einer etwa 0,5 mm höheren von der Messkammer abgewandten Wandung (Außenwandung 17) und einem Boden 18 eine Nut 19 bildet, die um die Messkammer 7 herum verläuft.

Zur Befestigung der Membran 11 wird ein am Rand der Membran 11 umlaufende Wulst 20, der sich auf der der Messkammer 7 zugewandten Seite der Membran 11 befindet, in die Nut 19 gedrückt. Der Wulst 20 der Membran 11 kann eine Breite von ca. 2 mm gemessen in Längserstreckung der Membran 11 aufweisen. Zweckmäßig weist der ebene Bereich der Membran 11 innerhalb des Wulstrandes 20 einen Durchmesser von wenigstens 12 mm auf, bei einer geeigneten Dicke in diesem Bereich von etwa 0,4 mm bis etwa 0,5 mm.

Die Dimensionierung der Innenwand 16 bezogen auf die Tiefe der Nut 19 wird dabei so vorgenommen, dass sich nach Eindrücken des Wulstes 20 in die Nut 19 der flache Bereich 21 der Membran durch einen minimalen Spalt von der Innenwand 16 abhebt. Bei der Koppelung des Druckdomes 1 mit dem Transducer 12 schließt sich der Spalt ohne die Membran 11 nennenswert zu deformieren, wodurch ein Aufwölben der Membran 11 vermieden wird.

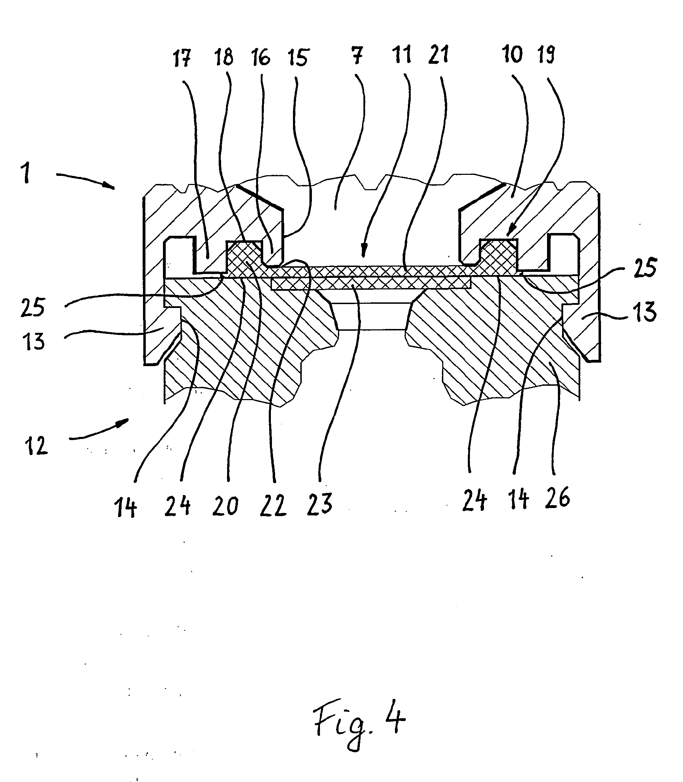

Der Höhenunterschied der Außenwand 17 gegenüber der Innenwand 16 ist geringer als die durchschnittliche Dicke des flachen Bereiches 21 der Membran. Die Höhe der Außenwandung 17 wird dabei so festgelegt, dass die Membran 11 im Bereich des Wulstes 20 nach Einsetzen des Wulstes 20 in die Nut 19 wenigstens etwa 0,1 mm, vorzugsweise zumindest etwa 0,3 mm über die Außenwandung 17 der Nut 19 herausragt. Bei dem Aufsetzen des Druckdomes 1 auf den Transducer 12 wird die Membran 11 im Bereich des Wulstes 20 leicht gequetscht, wodurch sich im Bereich des Spaltes 22, der zwischen der Außenwandung 17 und dem entsprechenden Widerlager an dem Transducer 12, dem die Transducermembran 23 umgebenden Bereich 24, entsteht, eine Art Dichtlippe 25 gegen von außen eindringende Luft bildet (Fig. 4).

Für den Einsatz des erfindungsgemäßen Verbindungselementes in blutführenden Systemen, z.B. bei der Dialyse, ist der Eintrittskanal und/oder der Austrittskanal 5 zu einer zur Membran 11 parallelen Ebene geneigt angeordnet. Vorteilhaft ist es dabei, wenn die Neigung des Eintrittskanals 3 und/oder des Austrittskanals 5 zu einer zur Membran 11 parallelen Ebene etwa 15° bis 45°, vorzugsweise 15° bis 30°, besonders bevorzugt etwa 20° beträgt. Dadurch können die Kräfte auf in der Flüssigkeit vorhandenen Zellen beim Durchströmen des Verbindungselementes minimiert werden. So lässt sich das Risiko einer Hämolyse, das heißt, ein Platzen der roten Blutkörperchen, allein durch die Strömung in dem Druckdom weitestgehend vermeiden.

Das in Fig. 2 dargestellte Systemelement zur lösbaren abgedichteten Verbindung eines Messwertaufnehmers mit einem Fluidsystem in Form eines Transducers 12 enthält wenigstens einen nicht dargestellten Sensor zur Umwandlung von Drücken und Druckänderungen in elektrische Signale, wobei der Sensor in einem Gehäuse 26 angeordnet ist und zumindest ein Teil des Gehäuses 26 mit einem Druck übertragenden Fluid oder Gel gefüllt ist, und das Gehäuse 26 an einer Seite mit einer Transducermembran 23 abgeschlossen ist, wobei das Gehäuse 26 ferner im die Transducermembran 23 umgebenden Bereich 24 zur Anlage eines weiter oben beschriebenen Druckdomes 1 ausgebildet ist, wobei der umgebende Bereich 24 die Transducermembran 23 im wesentlichen ringförmig umschließt, und der Messbereich des Sensors wenigstens Differenzdrücke gegenüber der Umgebung von -250 mm Hg bis +1000 mm Hg umfasst, möglichst bis zu Differenzdrücken gegenüber der Umgebung von -350 mm Hg. Bevorzugt umfasst der Messbereich des Sensors wenigstens Differenzdrücke gegenüber der Umgebung von -400 mm Hg bis +3000 mm Hg.

Der umgebende Bereich 24 ist annähernd technisch eben und die Oberfläche des umgebenden Bereiches 24 ist im wesentlichen kratzerfrei geschlichtet, vorzugsweise feingedreht, geschliffen oder poliert. Die Oberfläche des umgebenden Bereiches 24 sollte eine gemittelte Rauhtiefe R_{z} von nicht mehr als 20 *µ*m (z.B. nach DIN 4768 T 1) und/oder eine maximale Rauhtiefe Rₘₐₓ von nicht mehr als 30 µm, vorzugsweise nicht mehr als etwa 20 µm aufweisen.

Die für die Messung negativer Drücke erforderliche Dichtwirkung läßt sich besonders sicher erhalten, wenn der umgebende Bereich 24 eine Breite von wenigstens 2 *µ*m aufweist.

Beim Zusammensetzen von Druckdom 1 und Transducer 12 (Fig. 3) läßt sich der Einschluß auch kleinster Luftpolster weitgehend vermeiden, da die Transducermembran 23 gegenüber dem sie umgebenden Bereich 24 geringfügig vorgewölbt ist, z.B. nicht mehr als 1 mm, vorzugsweise nicht mehr als 0,6 mm.

Die Transducermembran 23 kann aus einem RTV-Silicon gebildet sein. Es kann zur Verbesserung der Langzeitstabilität aber auch vorteilhaft sein, wenn die Transducermembran 23 aus einem Polyurethan, vorzugsweise einem TPE-Polyurethan gebildet ist.

Eine Koppeleinrichtung zur mechanischen Verbindung des Druckdomes 1 und des Transducers 12 kann durch die Haken 13 und eine korrespondierende Hinterschneidung 14 am Transducer 12 gebildet sein. Es kommen aber auch andere bekannte Koppelmechanismen in Frage, wobei jedoch eine rotierende Bewegung zwischen Membran 11 und Transducermembran 23 vermieden werden sollte. Dazu können beispielsweise Haken und Hinterschneidung vertauscht werden. Auch kann eine andere Schnappverbindung vorgesehen werden, z.B. wie in WO 99/37983 vorgeschlagen. Weiterhin kommen Bajonettverbindungen (Renkverbindungen) vorzugsweise mit einem Überwurfring in Frage, auch entsprechend als Verschraubung, wenn ein übermäßiges Anziehen der Verbindung durch geeignete Abstandhalter oder dergleichen verhindert wird. Schließlich können noch schwenkbar montierte Klammern oder Kniehebelverschlüsse nach Art von Schließhaken vorgesehen werden.

Bei erfolgter Koppelung (vgl. Fig. 4) wird die Membran 11 des Druckdomes 1 im Bereich des Wulstes 20 gegen den die Transducermembran 23 umgebenden Bereich 24 gedrückt, so dass eine luftdichte Abdichtung zwischen der Membran 11 des Druckdomes 1 und dem Transducer 12 erfolgt, wobei bei erfolgter Koppelung die Membran 11 des Druckdomes 1 im Bereich der Wulst 20 mit wenigstens 50 N, vorzugsweise um 90-110 N gegen den die Transducermembran 23 umgebenden Bereich 24 gedrückt wird, und/oder nach erfolgter Koppelung die Membran 11 des Druckdomes 1 im Bereich der Wulst 20 etwa um 0,1 mm bis etwa 0,3 mm zusammengedrückt ist.

## Patentansprüche

1. Systemelement zur lösbaren abgedichteten Verbindung eines Meßwertaufnehmers mit einem Fluidsystem, mit
einer mit dem Fluidsystem durchströmbar verbindbaren Messkammer (7), wobei die Messkammer (7) in einem Gehäuse (10) gebildet ist und ein Teil der Wandung der Messkammer (7) durch eine Membran (11) gebildet ist, die wesentlich nachgiebiger ist, als der übrige Teil der Wandung (15) der Messkammer (7),
**dadurch gekennzeichnet, dass**
die Membran (11) einen umlaufenden Wulst (20) aufweist, der sich auf der der Messkammer (7) zugewandten Seite der Membran (11) befindet, wobei der Wulst (20) in eine in dem Gehäuse (10) gebildete Nut (19) eingreift, die um die Messkammer (7) verläuft und die der Messkammer (7) zugewandte Wandung (16) der Nut (19) eine geringere Höhe aufweist, als die von der Messkammer (7) abgewandte Wandung (17) der Nut (19), wobei die Membran (11) im Bereich des Wulstes (20) nach Einsetzen des Wulstes (20) in die Nut (19) wenigstens 0,1 mm, vorzugsweise zumindest 0,3 mm über die Außenwandung (17) der Nut (19) herausragt.

2. Systemelement nach Anspruch 1.
**dadurch gekennzeichnet, dass**
der Höhenunterschied der Außenwandung (17) gegenüber der Innenwandung (16) der Nut (19) geringer ist als die durchschnittliche Dicke der Membran (11) im Bereich (21) innerhalb des umlaufenden Wulstes (20).

3. Systemelement nach einem der vorhergehenden Ansprüche,
**dadurch gekennzeichnet, dass**
die Membran (11) aus einem Ethylen-Propylen-Dien-Kautschuk hergestellt ist.

4. Systemelement nach einem der Ansprüche 1 bis 2,
**dadurch gekennzeichnet, dass**,
die Membran (11) aus einem Thermoplastischen Polyurethan-Elastomer hergestellt ist.

5. Systemelement nach Anspruch 4,
**dadurch gekennzeichnet, dass**
die Membran (11) aus einem Thermoplastischen Polyurethan-Elastomer der Klasse Styrol-Ethylen-Butylen-Styrol-Copolymer hergestellt ist.

6. Systemelement nach einem der vorhergehenden Ansprüche,
**dadurch gekennzeichnet, dass**
die Membran (11) bei einem Anpressdruck von 60 N gegen eine feingedrehte stählerne Oberfläche einen Unterdruck von 530 hPa gegen Luft dichte.

7. Systemelement nach einem der vorhergehenden Ansprüche,
**dadurch gekennzeichnet, dass**
die Membran (11) innerhalb des Wulstes (20) einen Durchmesser von wenigstens 12 mm aufweist.

8. Systemelement nach einem der vorhergehenden Ansprüche,
**dadurch gekennzeichnet, dass**
der Wulst (20) eine Breite von ca. 2 mm in Längserstreckung der Membran (11) aufweist.

9. Systemelement nach einem der vorhergehenden Ansprüche,
**dadurch gekennzeichnet, dass**
die Membran (11) innerhalb des Wulstes (20) eine Dicke von 0.4 mm bis 0.5 mm aufweist.

## Claims

1. A system element for the releasable sealed connection of a transducer to a fluid system, with a measuring chamber (7) which can be connected to the fluid system in such a way as to allow fluid to flow through, the measuring chamber (7) being formed in a housing (10) and part of the wall of the measuring chamber (7) being formed by a membrane (11) which is considerably more flexible than the rest of the wall (15) of the measuring chamber (7),
**characterized in that**
the membrane (11) has a peripheral bead (20) which is located on the side of the membrane (11) that faces toward the measuring chamber (7), the bead (20) engaging in a groove (19) which is formed in the housing (10) and extends around the measuring chamber (7) and the wall (16) of the groove (19) that faces toward the measuring chamber (7) being lower than the wall (17) of the groove (19) that faces away from the measuring chamber (7), whereas, after insertion of the bead (20) into the groove (19), the membrane (11) protrudes at least 0.1 mm, preferably at least 0.3 mm, beyond the outside wall (17) of the groove (19) in the area of the bead (20).

2. A system element as claimed in claim 1,
**characterized in that**
the difference in height of the outer wall (17) with respect to the inner wall (16) of the groove (19) is less than the average thickness of the membrane (11) in the area (21) within the peripheral bead (20).

3. A system element as claimed in one of the previous claims,
**characterized in that**
the membrane (11) is manufactured from an ethylene propylene diene caoutchouc.

4. A system element as claimed in one of the claims 1 to 2,
**characterized in that**
the membrane (11) is manufactured from a thermoplastic polyurethane elastomer.

5. A system element as claimed in claim 4,
**characterized in that**
the membrane (11) is manufactured from a thermoplastic polyurethane elastomer of the class styrene-ethylene/butylene-styrene copolymer.

6. A system element as claimed in one of the previous claims,
**characterized in that**
the membrane (11) seals a negative pressure of 530 hPa against air when pressed with a pressure of 60 N against a finely turned steel surface.

7. A system element as claimed in one of the previous claims,
**characterized in that**
the membrane (11) has within the bead (20) a diameter of at least 12 mm.

8. A system element as claimed in one of the previous claims,
**characterized in that**
the bead (20) has a width of approximately 2 mm in the longitudinal extent of the membrane (11).

9. A system element as claimed in one of the previous claims,
**characterized in that**
the membrane (11) has within the bead (20) a thickness of between 0.4 mm and 0.5 mm.

## Revendications

1. Élément de système pour la liaison étanchéifiée amovible d'un capteur avec un système de fluide, avec une chambre de mesure (7) pouvant être raccordée de façon traversante avec le système de fluide, la chambre de mesure (7) étant formée dans un boîtier (10) et une partie de la paroi de la chambre de mesure (7) étant formée par une membrane (11), laquelle est essentiellement plus flexible que le reste de la paroi (15) de la chambre d'essai (7),
**caractérisé en ce que**
la membrane (11) présente un bourrelet périphérique (20), lequel se trouve du côté de la membrane (11) tourné vers la chambre d'essai (7), le bourrelet (20) pénétrant dans une rainure (19) formée dans le boîtier (10), laquelle fait le tour de la chambre d'essai (7), et la paroi (16) de la rainure (19) tournée vers la chambre d'essai (7) présente une hauteur inférieure à la paroi (17) de la rainure (19) tournée du côté opposé à la chambre d'essai (7), la membrane (11) dépassant d'au moins 0,1 mm, de préférence d'au moins 0,3 mm la paroi extérieure (17) de la rainure (19), dans la région du bourrelet (2), après pose du bourrelet (20) dans la rainure (19).

2. Élément de système selon la revendication 1,
**caractérisé en ce que**
la différence de hauteur entre la paroi extérieure (17) et la paroi intérieure (16) de la rainure (19) est inférieure à l'épaisseur moyenne de la membrane (11) dans la région (21) à l'intérieur du bourrelet (20) périphérique.

3. Élément de système selon l'une des revendications précédentes,
**caractérisé en ce que**
la membrane (11) est fabriquée dans un caoutchouc éthylène-propylène-diène.

4. Élément de système selon l'une des revendications 1 à 2,
**caractérisé en ce que**
la membrane (11) est fabriquée dans un élastomère thermoplastique.

5. Élément de système selon la revendication 4,
**caractérisé en ce que**
la membrane (11) est fabriquée dans un élastomère thermoplastique de la classe copolymère styrène-éthylène-butylène-styrène.

6. Élément de système selon l'une des revendications précédentes,
**caractérisé en ce que**
la membrane (11) assure l'étanchéité par rapport à l'air d'une dépression de 530 hPa lors d'une pression d'appui de 60 N contre une surface en acier réalisée par tournage fin.

7. Élément de système selon l'une des revendications précédentes,
**caractérisé en ce que**
la membrane (11) présente un diamètre d'au moins 12 mm à l'intérieur du bourrelet (20).

8. Élément de système selon l'une des revendications précédentes,
**caractérisé en ce que**
le bourrelet (20) présente une largeur d'environ 2 mm dans la direction longitudinale de la membrane (11).

9. Élément de système selon l'une des revendications précédentes,
**caractérisé en ce que**
la membrane (11) présente une épaisseur de 0,4 mm à 0,5 mm à l'intérieur du bourrelet (20).
